# FASCICULE DE BREVET EUROPEEN

(11) **EP 1 484 037 B1**
(45) Date de publication et mention de la délivrance du brevet: **25.06.2014**
(21) Numéro de dépôt: 04290992.9
(22) Date de dépôt: 14.04.2004
(51) Int. Cl.: A61F 2/38

(54) **Prothèse postéro-stabilisée à plot fémoral anti-basculement**
Posterior stabilisierte Prothese mit einem Femurstift gegen Kippen
Postero-stabilised prosthesis with anti-tilting femoral stud

(30) Priorité: 24.04.2003 FR 0305063
(43) Date de publication de la demande: 08.12.2004
(73) Titulaire: Aesculap S.A.S., 52000 Chaumont (FR)
(72) Inventeur: Plumet, Sylvie, 52000 Chaumont (FR); Vouaux, Alexis, 52800 Poulangy (FR)
(74) Mandataire: Eidelsberg, Olivier Nathan

(56) Documents cités:
- EP-A- 0 381 352
- US-A- 4 209 861
- US-A- 5 147 405
- US-A- 5 824 100
- US-A- 5 879 392
- US-B1- 6 325 828

## Description

La présente invention se rapporte à une prothèse du genou postéro-stabilisée. Cette prothèse comporte une partie fémorale destinée à être fixée à un fémur, notamment par ancrage, et une partie tibiale, destinée à être fixée à la partie proximale d'un tibia, notamment également par ancrage. Entre la partie tibiale et la partie fémorale, il est prévu un insert (aussi appelé ménisque), qui peut être fixé à la partie tibiale ou mobile, usuellement en un matériau plus tendre que celui des parties fémorale et tibiale, tel que le polyéthylène. Dans sa partie supérieure, l'insert comporte des surfaces généralement concaves sur lesquelles viennent en contact de glissement ou roulement deux condyles issus de la partie fémorale. Un plot, dit plot tibial, fait saillie de la base de l'insert, notamment perpendiculairement. Entre les deux condyles de la partie fémorale, il est formé une ouverture, dite espace inter-condyles, dans lequel pénètre le plot tibial. Pour délimiter cette ouverture, du côté postérieur, il est prévu un plot dit fémoral, s'étendant transversalement au plot tibial, d'un condyle à l'autre.

Lors de la rotation ou flexion du genou, le plot fémoral, en général à partir d'un angle de flexion d'environ 30°, vient en contact avec le plot tibial.

Les caractéristiques des préambules des revendications 1 et 8 sont connues du document US-A-5879392. Dans les prothèses actuelles, il existe, à partir de cette valeur de contact de 30° de flexion un recul vers l'arrière ou postérieur de la partie fémorale qui permet d'obtenir une bonne flexion jusqu'à des valeurs de 120° à 130°, en particulier pour les personnes obèses. Sans ce recul postérieur, des grandes flexion de ce genre ne seraient pas possibles, en particulier pour les personnes obèses.

Cependant, au-delà de 80 à 90° de flexion, la partie fémorale présente un risque élevé de recul vers l'arrière, pouvant se traduire par des efforts importants et à terme par une dislocation de la prothèse.

En outre, l'insert au niveau du contact avec les condyles a tendance à s'user fortement.

La présente invention vise à surmonter ces inconvénients de l'art antérieur en proposant une prothèse du genou postéro-stabilisée, dont la partie fémorale a moins tendance à basculer, la prothèse du genou ayant une plus grande durée de vie, en particulier de son insert, et acceptant des grandes flexions de l'ordre de 120 à 130, voire 140°, même pour les personnes obèses.

Suivant l'invention, la prothèse totale du genou postéro stabilisée, constituée d'une partie fémorale comportant deux condyles entre lesquels s'étend un plot fémoral de forme cylindrique pour définir une ouverture, d'une partie tibiale comportant un plateau tibial, et d'un insert tibial disposé entre la partie fémorale et le plateau tibial, un plot tibial, issu d'une pièce de l'insert tibial, pénétrant dans l'ouverture et venant en contact avec le plot fémoral à partir d'un angle donné, notamment environ 20 à 30°, l'insert tibial ayant des surfaces supérieures concaves en contact avec les surfaces extérieures convexes des condyles, le contact définissant une zone de contact ayant un point central, est caractérisée en ce que la courbe délimitant la section en coupe transversale du plot fémoral est définie de sorte que le point central de la zone de contact subit une translation ou recul dans la direction postérieure, pour tout angle de flexion compris entre environ 20° à 30° jusqu'à 80 à 90°, et ne subit plus de recul postérieur pour tout angle compris entre environ 80 à 90° et une flexion maximale de 120-130°.

En prévoyant une telle cinématique pour le point de contact condyle-insert, c'est-à-dire une absence de recul postérieur de ce point pour des angles de flexion à partir de 80-90° jusqu'à flexion complète (120-135°), mais en ayant cependant un recul postérieur jusqu'à cette valeur de 80 à 90°, on diminue fortement le risque de recul et de basculement de la partie fémorale et l'usure de l'insert, tout en permettant cependant l'obtention de grandes flexions de la prothèse du genou, en particulier pour des personnes obèses. On obtient ainsi une prothèse de plus longue durée de vie, plus sûre et qui imite de manière plus précise la cinématique d'un genou naturel.

Suivant un mode de réalisation préféré, le recul postérieur est décroissant entre environ 20 à 30° et 80 à 90°, angle à partir duquel il devient nul.

Suivant la partie caractérisante de la revendication 8, la courbe délimitant la section en coupe transversale comporte au moins deux segments de forme convexe, notamment circulaire, se rejoignant en un point dit sommet, le point issu de la projection perpendiculaire du sommet sur un segment de droite reliant les deux segments convexes étant plus proche de l'extrémité postérieure du segment de droite, que de l'extrémité antérieure, suivant un rapport de 1/3 à 1/6.

Suivant un mode de réalisation préféré de l'invention, les segments de forme convexe de la courbe délimitant la section en coupe transversale du plot fémoral correspondent sensiblement à des segments de la courbe définie par la section transversale dans le plan antéro-postérieur ou sagittal de la surface extérieure des condyles, à une homothétie près.

Suivant un perfectionnement de l'invention, l'insert tibial de la prothèse, comportant un plot tibial faisant saillie, notamment perpendiculairement, de la base de l'insert, et ayant une face destinée à être tournée vers le côté postérieur, est caractérisé en ce que en coupe transversale dans le plan sagittal, la face postérieure définit une courbe, dite courbe de contact, qui a la forme d'une courbe concave ayant sa concavité tournée vers le côté postérieur, un point de la courbe, notamment le sommet de la courbe, à savoir le point le plus éloigné de la base, étant plus postérieur que d'autres points de la courbe, notamment la plupart des autres points de la courbe.

Dans cette prothèse, le plot fémoral roule ou glisse sur la face postérieure du plot tibial, au fur et à mesure que le genou fléchit. Dans les prothèses actuelles, le point de contact plot fémoral-plot tibial a tendance à monter (c'est-à-dire à s'éloigner de la base de l'insert) au fur et à mesure que le genou fléchit. En prévoyant ainsi un sommet de la courbe de contact plus postérieur, on combat un éventuel décrochage pour les grands angles de flexion et on obtient ainsi une prothèse plus sûre.

Suivant un mode de réalisation préféré de l'invention, la courbe de contact comporte un segment de droite, notamment dans sa partie intermédiaire entre la base et le sommet.

Suivant un perfectionnement de l'invention, la courbe, au moins en partie, est telle que à partir d'un point donné jusqu'au sommet, un point de la courbe est d'autant plus postérieur qu'il est proche du sommet.

Le point de contact plot fémoral-plot tibial aura tendance à descendre, au fur et à mesure de la flexion, diminuant ainsi le risque de basculement.

On décrit maintenant un mode de réalisation préféré de l'invention, donné uniquement à titre d'exemple, en se rapportant aux dessins, dans lesquels :
la figure 1 représente la partie fémorale d'une prothèse postéro-stabilisée sur un insert tibial destiné à être posé sur un plateau tibial, dans le plan dit antéro-postérieur, en position étendue du genou (flexion à 0°) ;

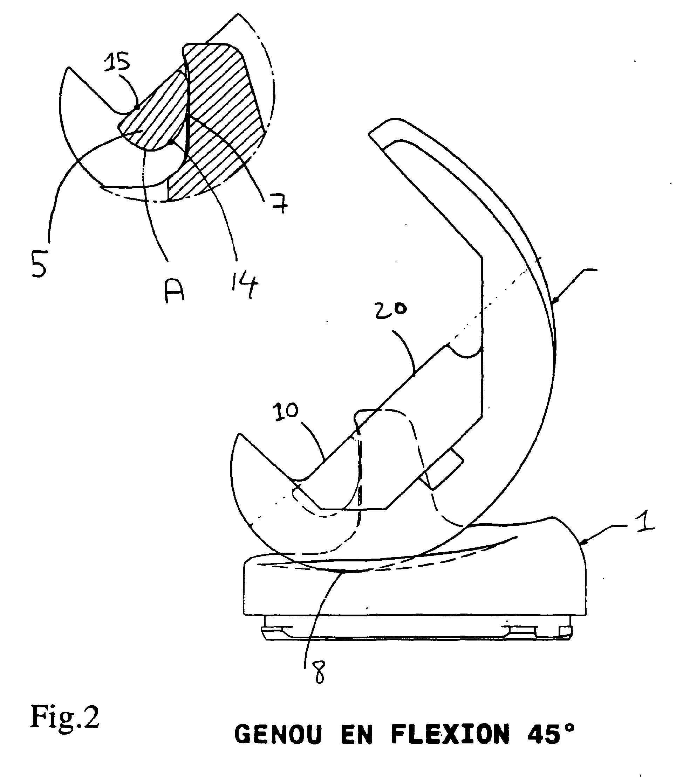

la figure 2 représente la partie fémorale de la figure 1, pour une flexion de 45° ;

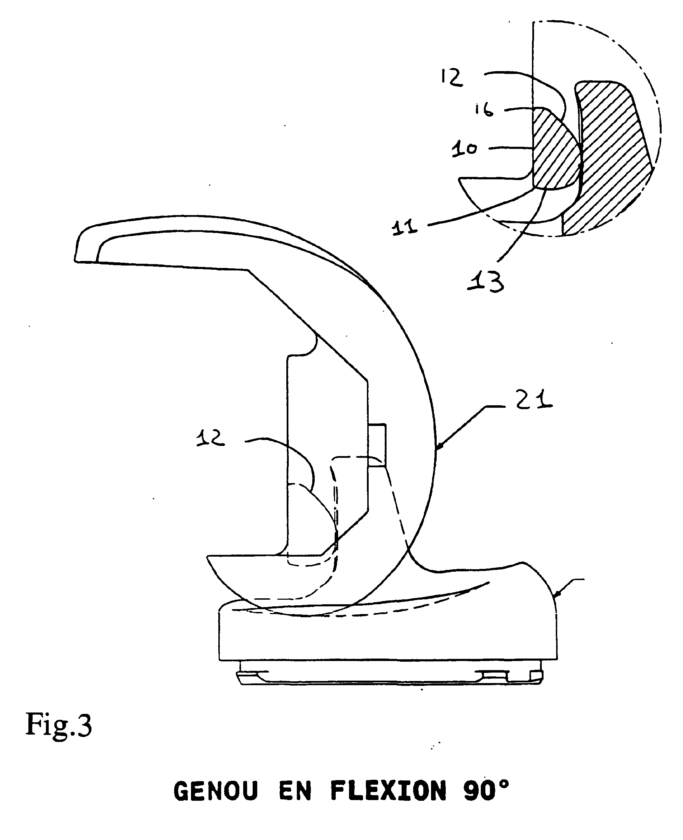

la figure 3 représente la partie fémorale de la figure 1, pour une flexion de 90° ;

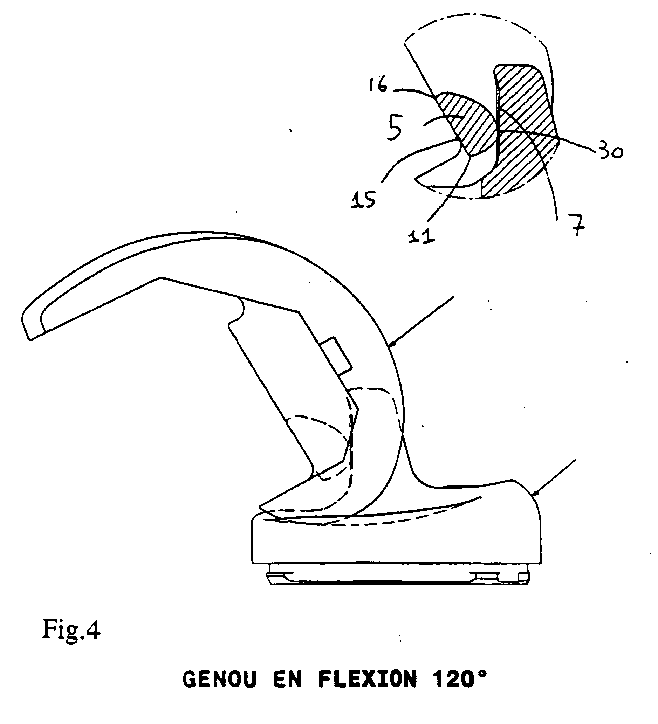

la figure 4 représente la partie fémorale de la figure 1, pour une flexion de 120°.

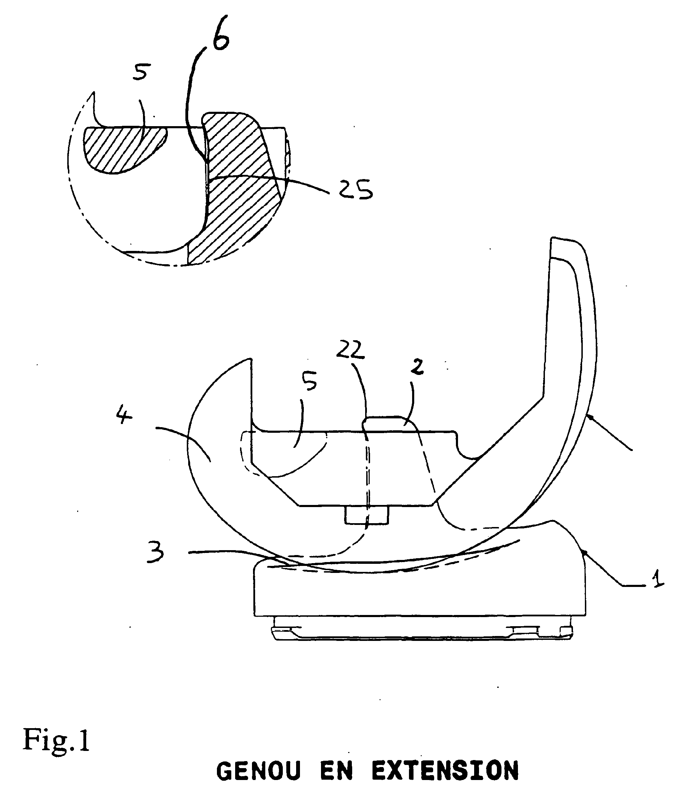

A la figure 1, il est représenté dans le plan antéro-postérieur, c'est-à-dire le plan défini par les axes longitudinaux du fémur et du tibia en flexion, un insert tibial 1 en polyéthylène, comportant un plot tibial 2 faisant saillie dans la direction verticale d'une base de l'insert, la base ayant deux surfaces 3 supérieures de contact avec lesquelles les surfaces extérieures des condyles 4 sont en contact.

Entre les deux condyles 4 droit et gauche (un seul étant représenté aux figures qui sont des vues en coupe), il est formé un espace inter-condyles traversé par le plot 2 tibial. Un plot fémoral 5 s'étend d'un condyle à l'autre dans la direction médio-latérale (perpendiculaire au plan des figures).

En position étendue du genou, le plot tibial et le plot fémoral sont à distance l'un de l'autre. A partir d'une flexion de 30°, le plot fémoral vient en contact sur une face 6 postérieure du plot tibial. Chaque condyle est en contact sur l'insert suivant une zone de contact, ayant un point central 8. Dans certains cas, en fonction des géométries relatives des surfaces en contact, cette zone peut être simplement un point, qui sera alors aussi le point central.

Suivant l'invention, on choisit la forme de la section transversale du plot fémoral, compte tenu de la forme des condyles, des surfaces de contact de l'insert et de la face postérieure du plot tibial de sorte que le point 8 soit fixe pour tout angle de flexion compris entre 80 à 90° et 130°.

En partant d'une forme donnée des surfaces concaves supérieures de l'insert, d'une forme donnée des surfaces extérieures des condyles, de la forme de la face postérieure du plot tibial, on peut déterminer point par point la forme de la courbe de la section transversale du plot fémoral.

Ainsi dans le cas des géométries des différents éléments des figures, on a obtenu la courbe A.

Dans le cas où les condyles sont formés par deux surfaces circulaires, de rayons R1 et R2 (voir les figures), on obtient de bons résultats en prévoyant une courbe A ayant deux courbes circulaires de rayons R1/H et R2/H, où H est un coefficient d'homothétie, qui est fonction de la taille totale de la prothèse.

On peut ainsi prévoir que la section transversale du plot fémoral est constituée d'un segment 10 de droite ayant une extrémité 11 postérieure et une extrémité 16 antérieure à partir desquelles s'étendent deux segments de courbes 12 et 13, postérieure et antérieure. Les deux segments de courbes sont convexes, par exemple circulaires. Ils se rejoignent en un sommet 14. La projection 15 perpendiculaire du sommet 14 sur la droite 10 se trouve plus proche de l'extrémité postérieure 11 que de l'extrémité antérieure 16. En particulier, le rapport de la distance de 15 à 11 sur la distance de 15 à 16 est compris entre 1/3 et 1/6.

En particulier, comme on peut le voir aux figures la forme de la courbe fermée 10-12-13 correspond, à une homothétie près, à la forme de la surface 21 extérieure des condyles en dessous de l'horizontale en position d'extension du genou complétée par un segment horizontal 20 (en partie en pointillés à la figure).

Le plot tibial comporte à son sommet une sorte de bec 22 faisant saillie dans la direction du plot fémoral. La surface postérieure du plot tibial a la forme d'une cuvette dont le fond 25 est sensiblement plat et s'étend sur sensiblement toute l'étendue verticale du plot.

Comme on le voit aux figures en outre, la forme de la surface postérieure du plot tibial est choisie de sorte que le point de contact 30 plot fémoral-plot tibial s'abaisse au fur et à mesure que l'angle de flexion augmente. On diminue ainsi encore plus le risque de basculement de la prothèse.

## Revendications

1. Prothèse totale du genou postéro-stabilisée, constituée d'une partie fémorale comportant deux condyles entre lesquels s'étend un plot fémoral (5) de forme cylindrique pour définir une ouverture, d'une partie tibiale comportant un plateau tibial et d'un insert tibial (1) disposé entre la partie fémorale et le plateau tibial, un plot tibial (2), issu d'une pièce de l'insert tibial, pénétrant dans l'ouverture et venant en contact avec le plot fémoral à partir d'un angle donné, notamment compris entre environ 20 et 30°, l'insert tibial ayant des surfaces supérieures concaves en contact avec les surfaces extérieures convexes des condyles, le contact définissant une zone de contact ayant un point central, est **caractérisée en ce que** la courbe délimitant la section en coupe transversale du plot fémoral est définie de sorte que le point central de la zone de contact subit une translation ou recul dans la direction postérieure, pour tout angle de flexion compris entre le dit angle donné jusqu'à 80 à 90°, et ne subit plus de recul postérieur pour tout angle compris entre environ 80 à 90° et une flexion maximale de 120-130°.

2. Prothèse totale du genou suivant la revendication 1, **caractérisée en ce que** le recul postérieur est décroissant entre environ 20 à 30° et 80 à 90°, angle à partir duquel il devient nul.

3. Prothèse totale du genou suivant la revendication 1 ou 2, **caractérisée en ce que** la courbe délimitant la section en coupe transversale comporte deux segments (12, 13) de forme convexe, notamment des arcs de cercle, se rejoignant en un point (14) dit sommet, le point issu de la projection perpendiculaire du sommet sur un segment de droite reliant les deux segments étant plus proche de l'extrémité postérieure du segment de droite.

4. Prothèse totale du genou suivant la revendication 1, 2 ou 3, **caractérisée en ce que** les segments (12, 13) de forme convexe de la courbe délimitant la section en coupe transversale du plot (5) fémoral correspondent sensiblement à des segments de la courbe définie par la section transversale dans le plan antéro-postérieur de la surface (21) extérieure des condyles.

5. Prothèse suivant l'une des revendications 1 à 4, **caractérisée en ce que** l'insert (1) tibial de la prothèse, comporte un plot (2) tibial faisant saillie, notamment perpendiculairement, de la base de l'insert, et a une face (6) destinée à être tournée vers le côté postérieur, en coupe transversale dans le plan sagittal, ladite face (6) postérieure définit une courbe, dite courbe de contact, qui a la forme d'une courbe concave ayant sa concavité tournée vers le côté postérieur, le sommet de la courbe, à savoir le point le plus éloigné de la base, étant plus postérieur que d'autres points de la courbe, notamment la plupart des autres points de la courbe.

6. Prothèse suivant l'une des revendications 1 à 5, **caractérisée en ce que** la courbe de contact comporte un segment de droite, notamment dans sa partie intermédiaire entre la base et le sommet.

7. Prothèse suivant l'une des revendications précédentes, **caractérisée en ce que** la courbe, au moins en partie, est telle que à partir d'un point donné jusqu'au sommet, un point de la courbe est d'autant plus postérieur qu'il est proche du sommet.

8. Partie fémorale d'une prothèse du genou postéro-stabilisée comportant deux condyles entre lesquels est défini un espace inter-condyles délimité du côté postérieur par un plot (5) fémoral s'étendant dans la direction médio-latérale, **caractérisée en ce que** la courbe délimitant la section en coupe transversale comporte deux segments (12, 13) de forme convexe, notamment des arcs de cercle, se rejoignant en un point (14) dit sommet, le point issu de la projection perpendiculaire du sommet sur un segment de droite reliant les deux segments étant plus proche de l'extrémité postérieure du segment de droite, que de l'extrémité antérieure, suivant un rapport de 1/3 à 1/6.

9. Partie fémorale suivant la revendication 8, **caractérisée en ce que** les segments (12, 13) de forme convexe, notamment des arcs de cercle, de la courbe délimitant la section en coupe transversale du plot (5) fémoral correspondent sensiblement à des segments de la courbe définie par la section transversale dans le plan antéro-postérieur ou sagittal de la surface (21) extérieure des condyles (4), à une homothétie près.

10. Partie fémorale suivant la revendication 8 ou 9, **caractérisée en ce que** les segments (12, 13) de forme convexe, notamment des arcs de cercle, de la courbe délimitant la section en coupe transversale du plot (5) fémoral correspondent sensiblement à des segments de la courbe définie par la section transversale dans le plan antéro-postérieur ou sagittal de la surface (21) extérieure des condyles (4), à une homothétie près.

## Patentansprüche

1. Posterior stabilisierte Knietotalprothese, bestehend aus einem Femurteil, der zwei Kondylen umfasst, zwischen denen ein Femurstift (5) zylindrischer Form verläuft, um eine Öffnung zu definieren, aus einem Tibiateil, der eine Tibiaplatte umfasst, und aus einem Tibiaeinsatz (1), der zwischen dem Femurteil und der Tibiaplatte angeordnet ist, wobei ein Tibiastift (2), der aus einem Stück des Tibiaeinsatzes hervorgegangen ist, in die Öffnung eindringt und ab einem vorgegebenen Winkel, insbesondere zwischen etwa 20 und 30°, mit dem Femurstift in Kontakt gelangt, wobei der Tibiaeinsatz konkave Oberflächen aufweist, die mit den konvexen Außenflächen der Kondylen in Kontakt sind, wobei der Kontakt einen Kontaktbereich mit einem Mittelpunkt definiert, und **dadurch gekennzeichnet, dass** die Kurve, welche den Querschnitt des Femurstiftes begrenzt, derart definiert ist, dass der Mittelpunkt des Kontaktbereiches bei jedem Beugungswinkel im Bereich zwischen dem vorgegebenen Winkel und 80 bis 90° ein(e) Verschiebung oder Zurückweichen in posteriorer Richtung erfährt und bei jedem Winkel im Bereich zwischen etwa 80 bis 90° und einer maximalen Beugung von 120 bis 130° kein posteriores Zurückweichen mehr erfährt.

2. Knietotalprothese nach Anspruch 1, **dadurch gekennzeichnet, dass** das posteriore Zurückweichen zwischen etwa 20 bis 30° und 80 bis 90°, Winkel, ab dem es null wird, abnehmend ist.

3. Knietotalprothese nach Anspruch 1 oder 2, **dadurch gekennzeichnet, dass** die den Querschnitt begrenzende Kurve zwei Segmente (12, 13) konvexer Form, insbesondere Kreisbögen umfasst, die in einem sogenannten Scheitelpunkt (14) zusammenlaufen, wobei der aus der senkrechten Projektion des Scheitels auf ein die beiden Segmente verbindendes Geradensegment hervorgegangene Punkt dem posterioren Ende des Geradensegments näher gelegen ist.

4. Knietotalprothese nach Anspruch 1, 2 oder 3, **dadurch gekennzeichnet, dass** die Segmente (12, 13) konvexer Form der Kurve, welche den Querschnitt des Femurstiftes (5) begrenzt, im Wesentlichen Segmenten der Kurve entsprechen, die durch den Querschnitt in der anterior-posterioren Ebene der Außenfläche (21) der Kondylen definiert ist.

5. Prothese nach einem der Ansprüche 1 bis 4, **dadurch gekennzeichnet, dass** der Tibiaeinsatz (1) der Prothese einen Tibiastift (2), der insbesondere senkrecht von der Basis des Einsatzes vorspringt, umfasst und eine Fläche (6) aufweist, die dazu bestimmt ist, der posterioren Seite zugewandt zu werden, die posteriore Fläche (6), im Querschnitt in der Sagittalebene, eine Kurve, sogenannte Kontaktkurve definiert, welche die Form einer konkaven Kurve aufweist, deren Konkavität der posterioren Seite zugewandt ist, wobei der Scheitel der Kurve, nämlich der von der Basis am weitesten entfernt liegende Punkt, posteriorer ist als andere Punkte der Kurve, insbesondere die meisten anderen Punkte der Kurve.

6. Prothese nach einem der Ansprüche 1 bis 5, **dadurch gekennzeichnet, dass** die Kontaktkurve ein Geradensegment, insbesondere in ihrem Zwischenteil zwischen der Basis und dem Scheitel umfasst.

7. Prothese nach einem der vorhergehenden Ansprüche, **dadurch gekennzeichnet, dass** die Kurve, wenigstens teilweise, derart ist, dass ab einem vorgegebenen Punkt bis zum Scheitel ein Punkt der Kurve umso mehr posterior ist, als er nahe dem Scheitel liegt.

8. Femurteil einer posterior stabilisierten Knieprothese, umfassend zwei Kondylen, zwischen denen ein interkondylärer Raum definiert ist, welcher auf der posterioren Seite durch einen Femurstift (5), der sich in mediolateraler Richtung erstreckt, begrenzt ist, **dadurch gekennzeichnet, dass** die den Querschnitt begrenzende Kurve zwei Segmente (12, 13) konvexer Form, insbesondere Kreisbögen umfasst, die in einem sogenannten Scheitelpunkt (14) zusammenlaufen, wobei der aus der senkrechten Projektion des Scheitels auf ein die beiden Segmente verbindendes Geradensegment hervorgegangene Punkt dem posterioren Ende des Geradensegments in einem Verhältnis von 1/3 bis 1/6 näher gelegen ist als dem anterioren Ende.

9. Femurteil nach Anspruch 8, **dadurch gekennzeichnet, dass** die Segmente (12, 13) konvexer Form, insbesondere Kreisbögen, der Kurve, welche den Querschnitt des Femurstiftes (5) begrenzt, bis auf eine Homothetie im Wesentlichen Segmenten der Kurve entsprechen, die durch den Querschnitt in der anterior-posterioren oder Sagittal-Ebene der Außenfläche (21) der Kondylen (4) definiert ist.

10. Femurteil nach Anspruch 8 oder 9, **dadurch gekennzeichnet, dass** die Segmente (12, 13) konvexer Form, insbesondere Kreisbögen, der Kurve, welche den Querschnitt des Femurstiftes (5) begrenzt, bis auf eine Homothetie im Wesentlichen Segmenten der Kurve entsprechen, die durch den Querschnitt in der anterior-posterioren oder Sagittal-Ebene der Außenfläche (21) der Kondylen (4) definiert ist.

## Claims

1. Full postero-stabilised knee prosthesis, formed by a femoral part comprising two condyles between which extends a femoral stud (5) of a cylindrical shape in order to define an opening, by a tibial part having a tibial plate, and by a tibial insert (11) disposed between the femoral part and the tibial plate, a tibial stud (2) projecting from a piece of the tibial insert, passing into the opening and coming into contact with the femoral stud from a given angle, in particular between about 20 and 30°, the tibial insert having concave upper surfaces in contact with the convex outer surfaces of the condyles, the contact defining a contact zone having a central point, is **characterised in that** the curve delimiting the transverse cross-section of the femoral stud is defined so that the central point of the contact zone undergoes a translation or recoil in the posterior direction for any angle of flexion between the said given angle up to 80 to 90°, and no longer undergoes any posterior recoil for any angle between about 80 to 90° and a maximum flexion of 120-130°.

2. Full knee prosthesis as claimed in claim 1, **characterised in that** the posterior recoil decreases between about 20 to 30° and 80 to 90°, an angle from which it becomes zero.

3. Full knee prosthesis as claimed in claim 1 or 2, **characterised in that** the curve delimiting the transverse cross-section has two segments (12, 13) which are convex, in particular arcs of a circle, meeting at a so-called summit point (14), the point formed by the perpendicular projection from the summit to a straight segment connecting the two convex segments being closer to the posterior end of the straight segment.

4. Full knee prosthesis as claimed in claim 1, 2 or 3, **characterised in that** the convex segments (12, 13) of the curve delimiting the transverse cross-section of the femoral stud (5) correspond substantially to segments of the curve defined by the transverse cross-section in the antero-posterior plane of the outer surface (21) of the condyles.

5. Prosthesis as claimed in any one of claims 1 to 4, **characterised in that** the tibial insert (1) of the prosthesis has a tibial stud (2) projecting, in particular in the perpendicular direction, from the base of the insert, and has a face (6) intended to be turned towards the posterior side, in transverse cross-section in the sagittal plane, said posterior face (6) defines a curve, a so-called contact curve, which is in the from of a concave curve with its concavity turned towards the posterior side, the summit of the curve, i.e. the point furthest from the base, being more posterior than other points on the curve, in particular most of the other points on the curve.

6. Prosthesis as claimed in any one of claims 1 to 5, **characterised in that** the contact curve has a straight segment, in particular in its intermediate part between the base and the summit.

7. Prosthesis as claimed in any one of the preceding claims, **characterised in that** the curve is at least partially of such a shape that from a given point up to the summit a point on the curve is more posterior the closer it is to the summit.

8. Femoral part of a postero-stabilised knee prosthesis having two condyles between which an inter-condyle space is defined, delimited on the posterior side by a femoral stud (5) extending in the medio-lateral direction, **characterised in that** the curve delimiting the transverse cross-section has two segments (12, 13) which are convex, in particular arcs of a circle, meeting at a so-called summit point (14), the point formed by the perpendicular projection from the summit to a straight segment connecting the two segments being closer to the posterior end of the straight segment than to the anterior end, in particular by a ratio of 1/3 to 1/6.

9. Femoral part as claimed in claim 7, **characterised in that** the point formed by the perpendicular projection from the summit to a straight segment connecting the two segments being closer to the posterior end of the straight segment than to the anterior end by a ratio of 1/3 to 1/6.

10. Femoral part as claimed in claim 8 or 9, **characterised in that** the segments (12, 13) which are convex, in particular arcs of a circle, of the curve delimiting the transverse cross-section of the femoral stud (5) correspond substantially to segments of the curve defined by the transverse cross-section in the antero-posterior or sagittal plane of the outer surface (21) of the condyles (4), to within a homothetic relationship.
